# EUROPEAN PATENT SPECIFICATION

(11) **EP 0 314 755 B1**
(45) Date of publication and mention of the grant of the patent: **24.08.1994**
(21) Application number: 88904835.1
(22) Date of filing: 11.05.1988
(51) Int. Cl.: A61K 7/06, A61K 31/30, A61K 37/02, C07K 5/08

(54) **Use of derivatives of GHL-Cu for the manufacture of a medicament for stimulating hair growth**
Gebrauch von GHL-Cu Derivaten für die Herstellung eines Medikaments zur Stimulierung des Haarwachstums
Utilisation de dérivés de GHL-Cu pour la préparation d'un médicament pour stimuler la croissance pilaire

(30) Priority: 11.05.1987 US 48444
(43) Date of publication of application: 10.05.1989
(73) Proprietor: PROCYTE CORPORATION, Kirkland, WA 98034-6900 (US)
(72) Inventor: PICKART, Loren, Ralph, Bellevue, WA 98006 (US)
(74) Representative: Brown, John David
(86) International application number: PCT/US88/01555
(87) International publication number: WO 88/08695

(56) References cited:
- EP-A- 0 189 182
- EP-A- 0 190 736
- WO-A-86/00222
- MERCK MANUAL OF DIAGNOSIS & THERAPY, 14th edition, 1982, Rahway, NJ (US); pp. 2051-2052#

## Description

### Technical Field

The present invention relates to cosmetic compositions and the stimulation of hair growth in general, and more specifically, to the use of derivatives of glycyl-L-histidyl-L-lysine: copper (II) (GHL-Cu) in the stimulation of hair growth in warm-blooded animals.

### Background Art

While attempts to grow hair date back approximately 5,000 years to ancient Egyptian formulas, and while in developed countries, approximately 50-100 million persons suffer from cosmetic hair loss, there has been relatively little significant progress in generating compositions and methods for stimulating the growth of hair. For instance, selected "hair growth" preparations which have been proposed include compositions of vitamins E, B₂, and B₆, crude drug extracts, karotin-solubilizing agents, germacides, and scalp-stimulating agents, all alleged to stimulate the growth of hair.

Another traditional treatment for the loss of hair has been hair transplantation. Briefly, plugs of skin containing hair are transplanted from areas of the scalp where the hair was growing to bald areas. This procedure is a costly one in addition to being time-consuming and relatively painful. Other non-drug approaches include ultra-violet radiation and exercise therapy.

Traditionally, one of the most common approaches to stimulating hair growth has been in the area of drug therapy. However, the use of drugs in this regard has met with limited success. One of the most promising compositions for stimulating the growth of hair is disclosed by Upjohn in U.S. Patent No. 4,596,812, which describes the use of a substance known as "Minoxidil", (Trade Mark). However, while the results generated through the use of Minoxidil (Trade Mark) have heretofore appeared promising, there is still a need in the art for improved compositions capable of stimulating the growth of hair in warm-blooded animals. The present invention fulfils this need, while further providing other related advantages.

EP-A-0190 736 discloses the use of a derivative of GHL-Cu in the enhancement of wound-healing processes.

### Disclosure of the Invention

According to the present invention there is provided use of a composition comprising a derivative of GHL-Cu having the general formula: 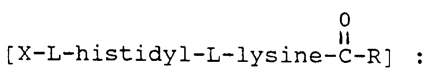
Copper (II) wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for stimulating the growth of hair in warm-blooded animals.

According to the present invention there is further provided use of a composition comprising a derivative of GHL-Cu having the general formula: 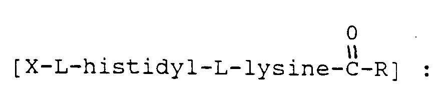
Copper (II) wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for increasing subcutaneous fat in a warm-blooded animal.

According to the present invention there is still further provided use of a composition comprising a derivative of GHL-Cu having the general formula: 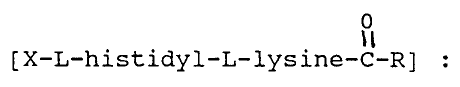
Copper (II) wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for increasing the density of hair follicles in warm-blooded animals.

In addition to the derivatives described above, other chemical modifications could be made to alter the biological activity of the derivatives of the present invention. For instance, glycine may be replaced by a variety of other small amino acids, including alanine, serine and valine. Further, the copper(II) binding affinity of the molecule could be increased by addition of an N-terminal amino acid such as glycine to convert glycyl-L-histidyl-L-lysine to glycyl-L-glycyl-L-histidyl-L-lysine. In addition, glycine could be added to a derivative as described above to create the corresponding tetrapeptide.

The compositions described herein may be injected intradermally or applied topically_{,} and are rendered suitable for administration to warm-blooded animals for the purposes of the present invention by combining the derivative with a vehicle which adapts the composition for either intradermal injection or topical application to a warm-blooded animal. Suitable vehicles include physiological saline.

### Brief Description of the Drawings

Figures 1 and 2 are photographs illustrating the stimulation of hair growth through the use of a representative medicament of the present invention.

Figure 3 is a microphotograph of a biopsy section taken through an area of enhanced hair growth. The photograph illustrates an increase in the density of hair follicles embedded in a heavy field of large, subcutaneous fat cells, as shown toward the right side of the figure.

Figure 4A is a photograph illustrating the stimulation of hair growth through the use, of another representative medicament of the present invention.

Figure 4B is a microphotograph of a biopsy section taken through an area of enhanced hair growth, generated by the derivative of Figure 4A. The photograph illustrates an increase in the density of hair follicles within the subcutaneous fat cell layer in the area near the injection site.

Figure 5 is a microphotograph of a control area (bottom) and an area of increased subcutaneous fat cells and hair follicle density (top) generated through use, of a representative medicament of the present invention.

Figure 6 is a photograph illustrating the stimulation of hair growth around a surgical defect utilizing a medicament of the present invention.

Figure 7 is a photograph illustrating enhanced hair growth surrounding a healing wound in a horse.

As described herein, various derivatives of GHL-Cu may be used to stimulate the growth of hair in warm-blooded animals. In addition, these derivatives can be tailored to increase their fat solubility, resulting in a form of the molecule which is more useful in a formulation of pharmaceutical and cosmetic creams and gels. The derivatives of the present invention are described in detail in EP-A-288278 and U.S. Patent No. 4,665,054, which documents are hereby incorporated by reference. The derivatives for use in the present invention may be prepared by esterification, by the removal of a water molecule, or by the addition of a group (either an alcohol such as octanol, methanol, benzol alcohol or NH₃) to the carboxylic acid terminus of GHL, resulting in the formation of the more lipophilic derivative. This increases fat solubility by (1) removal of the electric charge associated with the carboxylic acid group and (2) the introduction of hydrophilic groups into the molecule.

The overall chemical reaction in this transformation may be characterized as:

GHL - OH + R - H ---> GHL - R + H₂O.

In practice, the reaction is most readily carried out by adding the R group to the amino acid lysine prior to the combination of lysine with the other two amino acids to GHL. After the formation and isolation of GHL-R, the copper (II) is chelated to the molecule to form the bioactive complex.

The overall reaction to form the more lipophilic derivatives of GHL-Cu may be characterized:
1) lysine-OH + R-H -----> lysine-R + H₂O
2) lysine-R + blocked L-histidine -----> blocked L-histidine-L-lysine-R
3) blocked L-histidine-L-lysine-R + blocked-glycine -----> blocked glycyl-L-histidine-L-lysine-R
4) blocked glycyl-L-histidine-L-lysine-R -----> glycyl-L-histidine-L-lysine-R
5) glycyl-L-histidine-L-lysine-R + copper (II) -----> glycyl-L-histidine-L-lysine-R: copper (II).

Within preferred embodiments_{,} the derivative of GHL and copper are present in a 1:1 or 2:1 ratio.

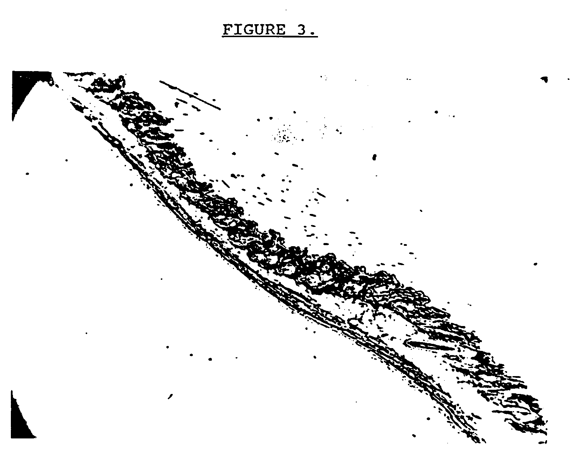

As noted above, the derivatives for use in the present invention are useful in stimulating the growth of hair in warm-blooded animals. While one of the characteristics associated with male pattern baldness is the severe diminution of hair follicles, use of the derivatives as described herein results in increased adipocyte formation, which is spatially and temporally linked with hair follicle formation, and is an integral phase of hair follicle formation (Hausman et al., Am. J. Anat. 161: 85-100, 1981). The results of the use of the derivatives as described herein are illustrated, in part, in Figures 3, 4B and 5, which are microphotographs of areas of enhanced hair growth.

The enhancement of subcutaneous fat in areas associated with increased hair growth is highly significant. Male pattern baldness is intimately associated with a dramatic reduction in the amount of subcutaneous fat associated with hair follicles that are nonproductive. Conversely, during periods of rapid hair growth in mammals, the subcutaneous fat content increase two- to threefold.

The derivatives for use in the present invention have clinical use in at least three primary areas: (1) the direct stimulation of hair growth in persons with hair loss, (2) the stimulation of hair transplants, and (3) increasing the subcutaneous fat content.

Within the present invention, it is generally preferred to administer the derivatives described herein intradermally in the center of the area to be treated, along with a suitable vehicle in a concentration of approximately 50 micrograms of derivative per .1 ml of vehicle. It is preferable to use a dosage of approximately 9 micrograms per cm² of area to be treated, although dosages greater than 9 micrograms/cm²_{,} up to approximately 40 micrograms/cm²_{,} may be used. Suitable vehicles in this regard include saline. When used in the form of a cream or gel and applied topically, it is useful to add a suitable penetrating agent, such as DMSO, to the composition. Suitable vehicles for use in cosmetic applications will be evident to those skilled in the art. For cosmetic purposes, the composition may further contain a moistening agent, softening agent, perfume or colorant.

To summarize the examples which follow, Example I illustrates the synthesis of glycyl-L-histidyl-L-lysine benzyl ester: copper(II). Example II demonstrates the syunthesis of glycyl-L-histidyl-L-lysine n-octyl ester: copper (II). Example III illustrates (A) the synthesis of glycyl-L-histidyl-L-lysine n-stearyl ester: copper(II), and (B) its synthesis by an alternative procedure. Based upon either procedure, one skilled in the art could substitute n-palmityl alcohol (16 carbons) for the n-stearyl alcohol (18 carbons) to yield glycyl-L-histidyl-L-lysine n-stearyl ester: copper (II). Example IV illustrates the synthesis of glycyl-L-histidyl-L-lysyl-L-prolyl-L-valyl-L-phenylalanyl-L-valine: copper(II) and glycyl-L-histidyl-L-lysyl-L-valyl-L-phenylalanyl-L-valine: copper (II). Examples V, VI and VII illustrate the hair growth stimulating activity of preferred medicaments of the present invention. Example VIII demonstrates the stimulation of hair growth around healing wounds in warm-blooded animals.

The following examples are offered by way of illustration.

### EXAMPLES

Sources of chemicals. Chemicals and peptide intermediates utilized in the following examples may be purchased from the following suppliers: Sigma Chemical Co. (St. Louis, Mo.); Peninsula Laboratories (San Carlos, Calif.); Aldridge Chemical Co. (Milwaukee, Wis.); Vega Biochemicals (Tucson, Ariz.); Pierce Chemical Co. (Rockford, Ill.); Research Biochemicals (Cleveland, Ohio); Van Waters and Rogers (South San Francisco, Calif.); Bachem, Inc. (Torrance, Calif.).

### EXAMPLE I

### Synthesis of glycyl-L-histidyl-L-lysine benzyl ester: copper (II)

N^{e}-benzyloxycarbonyl-L-lysine benzyl ester was dissolved in 1:1 hexane-ethyl acetate and coupled to N^{a}-t-butyloxycarbonyl-N^{im}-benzyloxycarbonyl-L-histidine using dicyclohexylcarbodiimide as a coupling agent. Sodium bicarbonate (10%) was added and the product extracted into the organic layer. The product, N^{a}-t-butyloxycarbonyl-N^{im}-benzyloxycarbonyl-L-histidyl-N^{e}-benzyloxycarbonyl-L-lysine benzyl ester, was crystallized from solution The N-terminal group of the blocked dipeptide was removed by stirring in 50% trifluoroacetic acid in dichloromethane for 30 minutes, then vacuum evaporated. The product, N^{im}-benzyloxycarbonyl-L-histidyl-N^{e}-benzoylcarbonyl-L-lysine benzyl ester, was coupled to benzyloxycarbonylglycine with dicyclohexylcarbodiimide as a coupling agent. Blocking groups were removed by catalytic hydrogenation using 10% palladium on carbon in glacial acetic acid. After lyophilization, the product, glycyl-L-histidyl-L-lysine benzyl ester, was dissolved in water and purified by ion-exchange chromatography on Dowex 50 X-4 cation-exchange resin and elution with 0.1 M ammonium hydroxide, the eluate being immediately neutralized with acetic acid. A further passage through an anion-exchange column BioRex 63 at neutral pH removed breakdown products with free carboxylic acid groups.

The glycyl-L-histidyl-L-lysine benzyl ester was dissolved in water with equimolar copper acetate added. The pH was raised to neutrality with sodium hydroxide. The solution was centrifuged at 20,000 x g for 1 hour at 3°C to remove poorly water-soluble material. The supernatant was lyophilized to obtain glycyl-L-histidyl-L-lysine benzyl ester: copper(II).

### EXAMPLE II

### Synthesis of glycyl-L-histidyl-L-lysine n-octyl ester: copper(II)

A mixture of N^{e}-benzyloxycarbonyl-L-lysine, n-octanol, benzene, and p-toluenesulfonic acid monohydrate was refluxed overnight using a Dean-Stark trap to remove water. After cooling, dry ethyl ether was added. The solution was then allowed to precipitate at 0°C overnight. A portion of the precipitated solid was added to 50 ml potassium carbonate solution and 50 ml dichloromethane. After extraction, the layers were separated and the organic phase washed with water and brine, then dried with anhydrous magnesium sulfate. Filtration, evaporation and purification by flash column chromatography gave n-octyl N^{e}-benzyloxycarbonyl-L-lysinate. The product was dissolved in tetrahydrofuran and mixed with N^{a}-t-butyloxycarbonyl-L-N^{im}-benzyloxycarbonyl-L-histidine, isobutyl chloroformate and N-methylmorpholine. After evaporation, water and ethyl acetate were added. The product was extracted into the organic phase, which was dried with anhydrous magnesium sulfate. Filtration, evaporation and purification by flash column chromatography gave n-octyl N^{a}-t-butyloxycarbonyl-N^{im} -benzyloxycarbonyl-L-histidyl-N^{e}-benzyloxycarbonyl-L-lysinate.

The product was dissolved in 50% trifluoroacetic acid in dichloromethane for 30 minutes, then evaporated, forming n-octyl N^{im}-benzyloxycarbonyl-L-histidyl-N^{e}-benzyloxycarbonyl-L-lysinate. This was dissolved in tetrahydrofuran, and isobutyl chloroformate, N-methylmorpholine and benzyloxycarbonylglycine were added to form n-octyl benzyloxycarbonylglycyl-N^{im}-benzyloxycarbonyl-L-histidyl-N^{e}-benzyloxycarbonyl-L-lysinate. This was dissolved in glacial acetic acid and hydrogenated overnight.

The resultant n-octyl ester of glycyl-L-histidyl-L-lysine was converted to the copper-complex by the addition of an equimolar quantity of copper diacetate. The pH was raised to neutrality with sodium hydroxide. The solution was centrifuged at 20,000 x g for 1 hour at 3°C to remove poorly water-soluble material. The supernatant was lyophilized to obtain glycyl-L-histidyl-L-lysine n-octyl ester: copper (II).

### EXAMPLE III

### A. Synthesis of glycyl-L-histidyl-L-lysine n-stearyl ester: copper (II)

A mixture of N^{e}-benzyloxycarbonyl-L-lysine, n-stearyl alcohol, benzene, and p-toluenesulfonic acid monohydrate was refluxed overnight using a Dean-Stark trap to remove water. After cooling, dry propyl ether was added to increase the total volume sixfold. The product was allowed to precipitate at 0°C overnight and filtered. A portion of the filtrate was added to 50 ml potassium carbonate and 50 ml dichloromethane. After extraction, the layers were separated, and the organic phase was washed with water and brine, then dried with anhydrous magnesium sulfate. Filtration, evaporation and purification by flash column chromatography gave n-stearyl N^{e}-benzyloxycarbonyl-L-lysinate. The product was dissolved in tetrahydrofuran and mixed with N^{a}-t-butyloxycarbonyl-N^{im}-benzyloxycarbonyl-L-histidine and isobutyl chloroformate and N-methylmorpholine. After evaporation, water and propyl acetate were added and the product was extracted into the organic phase, then dried with anhydrous magnesium sulfate. Filtration, evaporation and purification by flash column chromatography gave n-stearyl N^{a}-t-butyloxycarbonyl-N^{im}-benzyloxycarbonyl-L-histidyl-N^{e}-benzyloxycarbonyl-L-lysinate.

The product was dissolved in 50% trifluoroacetic acid in dichloromethane for 30 minutes, then evaporated, forming n-stearyl N^{im}-benzyloxycarbonyl-L-histidyl-N^{e}-benzyloxycarbonyl-L-lysinate, which was dissolved in tetrahydrofuran, isobutyl chloroformate, N-methylmorpholine and benzyloxycarbonylglycine to form n-stearyl benzyloxycarbonylglycyl-N^{im}-benzyloxycarbonyl-L-histidyl-N^{e}-benzyloxycarbonyl-L-lysinate. The product was dissolved in 50% trifluoroacetic acid in dichloromethane for 30 minutes, then evaporated, forming n-stearyl ester glycyl-L-histidyl-L-lysine.

The resultant molecule, glycyl-L-histidyl-L-lysine n-stearyl ester, was converted to the copper complex by the addition of an equimolar quantity of copper diacetate. The pH was raised to neutrality with sodium hydroxide to obtain a product useful for animal studies.

By substituting n-palmityl alcohol for the n-stearyl alcohol, glycyl-L-histidyl-L-lysine n-palmityl ester may be similarly synthesized.

### B. Alternative synthesis of glycyl-L-histidyl-L-lysine n-stearyl ester: copper (II)

N^{e}-benzyloxycarbonyl-L-lysine, n-stearyl alcohol, p-toluenesulfonic acid monohydrate, and benzene are refluxed together using a Dean-Stark trap to azeotropically remove the evolved water. After cooling to room temperature and then adding dry ethyl ether, n-stearyl N^{e}-benzyloxycarbonyl-L-lysinate p-toluenesulfonate salt is collected by filtration, treated with 2 M aqueous potassium bicarbonate solution, and extracted into dichloromethane. Evaporation gives the free amine, which is redissolved in dry tetrahydrofuran (THF) and added to a stirring solution of N^{a}-t-butyloxycarbonyl-N^{im}-benzyloxycarbonyl-L-histidine, N-methylmorpholine, and isobutyl chloroformate in dry THF at -15°C. The resulting fully protected dipeptide ester is treated with 1/1 trifluoroacetic acid/dichloromethane at room temperature, neutralized with saturated aqueous sodium bicarbonate solution, and extracted into ethyl acetate. Evaporation gives the partially deblocked dipeptide, which is redissolved in dry THF and added to a stirring solution of benzyloxycarbonyl-glycine, N-methylmorpholine and isobutyl chloroformate in dry THF at -15°C. The formed, fully protected tripeptide ester is totally deblocked by treatment with hydrogen gas in glacial acetic acid at room temperature in the presence of Pd-C catalyst. Filtration, evaporation and purification on a microcrystalline cellulose column followed by lyophilization give the desired tripeptide ester as its triacetate salt.

The resultant molecule, glycyl-L-histidyl-L-lysine n-stearyl ester, was converted to the copper-complex by the addition of an equimolar quantity of copper diacetate. The pH was raised to neutrality with sodium hydroxide to obtain a product useful for animal studies.

By substituting n-palmityl alcohol for the n-stearyl alcohol, glycyl-L-histidyl-L-lysine n-palmityl ester may be similarly synthesized.

### EXAMPLE IV

### Synthesis of glycyl-L-histidyl-L-lysyl-L-prolyl-L-valyl-L-phenylalanyl-L-valine: copper (II) and of glycyl-L-histidyl-L-lysyl-L-valyl-L-phenylalanyl-L-valine: copper (II)

These peptides are synthesized by standard solid-phase methods common to the peptide field (J. Stewart and J. Young, Solid Phase Peptide Synthesis, Pierce Chemical Co., 1984) Briefly stated, Boc-Val-O-Resin was sequentially coupled with other blocked amino acids using dicyclohexylcarbodiimide as a reaction agent. Protected amino acids, resins for solid-phase synthesis, and coupling agents were obtained from Peninsula Laboratories, San Carlos, California. Blocked amino acids are added in sequential order to obtain the desired peptide. The final peptide is deblocked using hydrogen fluoride. The final peptide is dissolved in 0.5% acetic acid and purified by passage through a Sephadex® G-15 column (Pharmacia). Addition of equimolar cupric acetate, followed by lyophilization, produces the active molecule.

### EXAMPLE V

### Use of glycyl-L-histidyl-L-lysine n-octyl ester: copper(II) to stimulate hair growth

### A. Stimulation of hair growth in normal skin

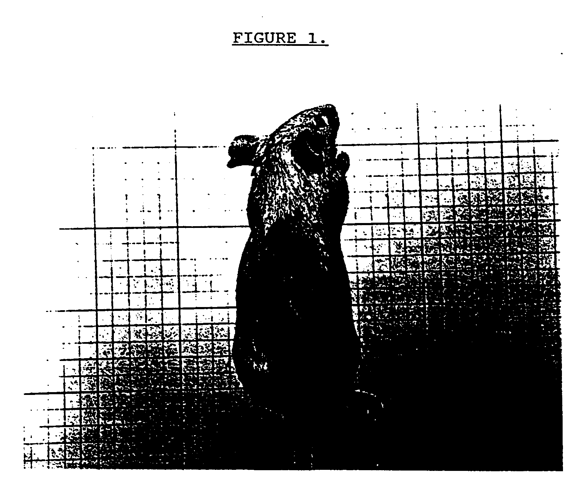

In order to demonstrate the stimulation of hair growth in warm-blooded animals, the backs of mice were shaved on day 1 using an electric shaver. Subsequently, a single dose of 50 micrograms of glycyl-L-histidyl-L-lysine n-octyl ester: Cu(II) was infiltrated under the skin in eight mice. As shown in Figure 1, by day 7 there was a markedly accelerated growth of hair around the injection area in all of the mice.

In some regions of enhanced hair growth, increased follicle densities were observed, along with increased amounts of subcutaneous fat.

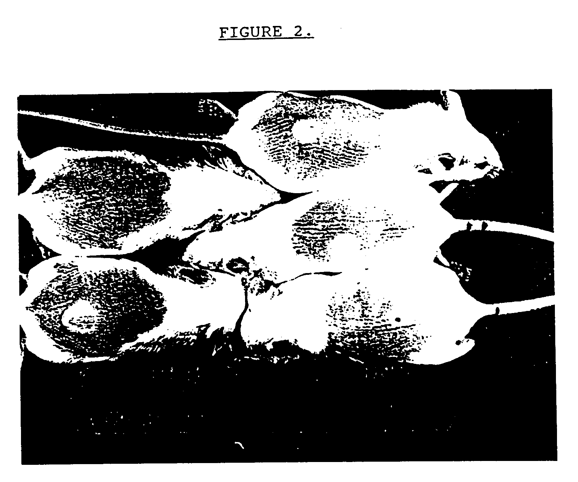

In another series of experiments, mice were shaved and injected once with Glycyl-L-Histidyl-L-Lysine Octyl Ester:Copper(II) at a dose of 500 micrograms per mouse. Figure 2 shows that there was a significant acceleration of hair growth within 2-3 weeks following the injection. Mice injected with saline did not show an acceleration.

In the region of accelerated hair growth, there was a significant increase in the thickness of the subcutaneous fat layer. It is known that the thickness of this fat layer is directly associated with hair growth. This increase in subcutaneous fat is shown by taking a biopsy sample at day 21 through the area and sectioning for histology slides. Figure 3 shows this increase in the fat layer. The injected area is on the right with the adjacent uninjected area on the left of the photograph. There was an increase in both the number and size of the fat cells. Measurements demonstrate that there was an approximately three-fold increase in the subcutaneous fat layer in the skin near the injection site.

These examples demonstrate that the stimulation of hair growth is observed both in normal skin and in newly healing regions utilizing the derivatives of the present invention.

### EXAMPLE VI

### Use of Glycyl-L-Histidyl-L-Lysine Decyl Ester Copper (II) to Stimulate Hair Growth in Mice

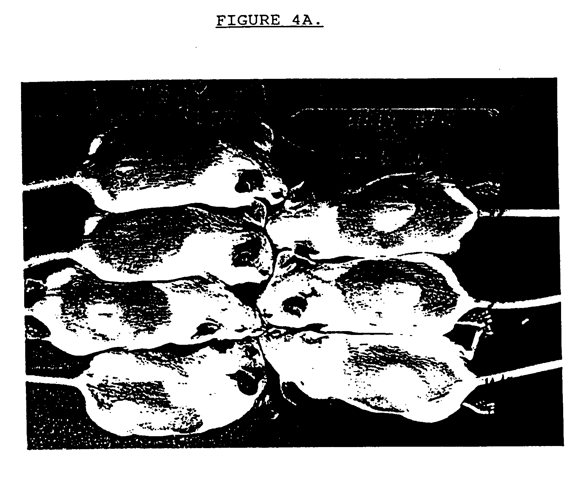

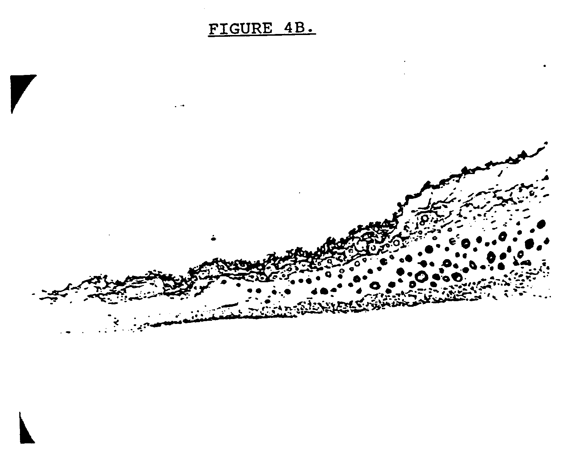

A group of ten mice were shaved and injected once with Glycyl-L-Histidyl-L-Lysine Decyl Ester : Copper (II) at a dose of 500 micrograms per mouse Figure 4A shows that there was a significant acceleration of hair growth within 2-3 weeks following the injection Microscopic examination also provided evidence of increased hair growth, fat cell layer, and increased hair follicle density in the area surrounding the injection site. Figure 4B shows that there was a marked proliferation of hair shaft units which appeared within the subcutaneous fat layer of the area of accelerated hair growth Examination of the skin distant from the injection site showed a normal histology.

### EXAMPLE VII

### Use of Glycyl-L-Histidyl-L-Lysine Palmityl Ester Copper(II) to Stimulate Hair Growth in Mice

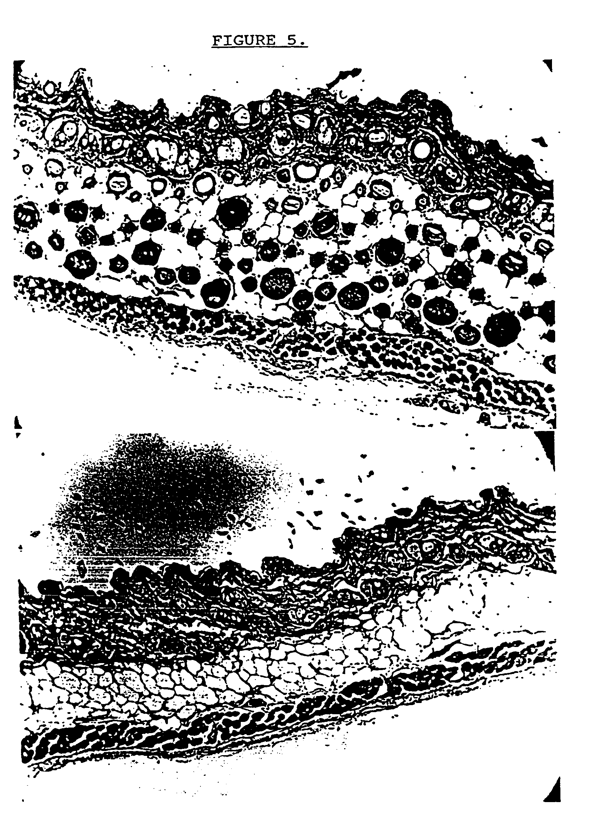

A group of ten mice were shaved and injected once with Glycyl-L-Histidyl-L-Lysine Palmityl Ester : Copper (II) at a dose of 500 micrograms per mouse. There was a significant acceleration of hair growth within 2-3 weeks following the injection. Histological sections through the area of accelerated hair growth were similar to those described in Example VI. This is shown in Figure 5. The photomicrograph demonstrates the large number of developing hair follicles within the subcutaneous fat layer, similar to that seen following the Glycyl-L-Histidyl-L-Lysine Decyl Ester:Cu injection.

### EXAMPLE VIII

### Stimulation of Hair Growth Around Healing Wounds

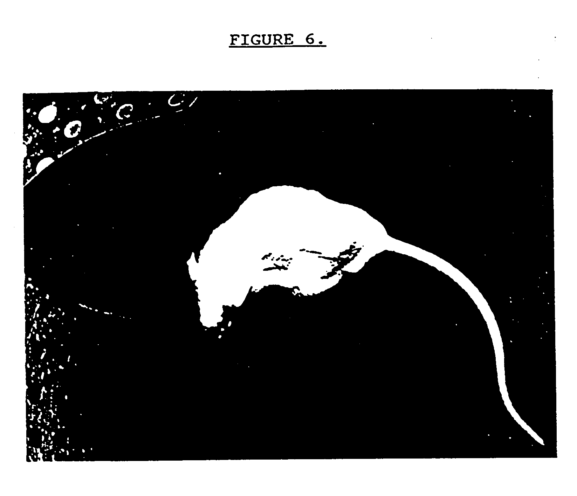

Re-establishment of hair growth is a normal part of healing. As shown in Figure 6, the compositions described herein may be used to stimulate the growth of hair around a surgical defect. In this model system, a 1.2 cm diameter full thickness defect was created on the backs of mice. Immediately following the surgery, the defect was treated with a solution containing Glycyl-L-Histidyl-L-Lysine Octyl Ester:Copper(II). The enhanced hair growth after healing was well advanced at 7 to 10 days and spread outward from the treated area (Figure 6). The maximal differential effect versus the control mice was observed approximately 22 days after treatment.

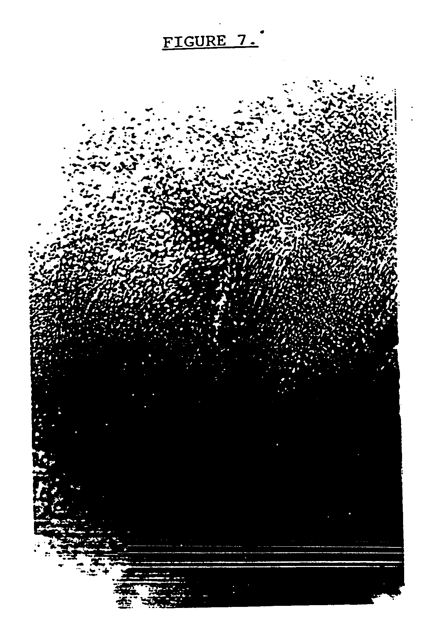

Treatment of a healing wound with Glycyl-L-Histidyl-L-Lysine:Copper(II) also causes an acceleration of hair growth. A horse with a deep, ulcerating wound was treated with a topical cream containing 0.4% Glycyl-L-Histidyl-L-Lysine:Copper(II) to accelerate the healing process. As part of the treatment protocol, the hair surrounding the wound was clipped to aid in cleansing the area. Treatment with the cream occurred every day until the wound was closing, followed by less frequent application. After 5 weeks, the wound was almost totally healed with only a small residual scab. As shown in Figure 7, there was a significant acceleration of hair growth surrounding the treated area when compared with the untreated areas.

## Claims

1. Use of a composition comprising a derivative of GHL-Cu having the general formula: 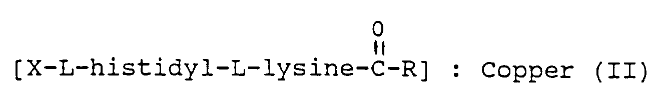 wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for stimulating the growth of hair in warm-blooded animals.

2. Use according to Claim 1 wherein the carbon portion of the alkoxy moiety is an unbranched chain.

3. Use according to Claim 2 wherein the unbranched chain is an n-octyl moiety.

4. Use according to Claim 1 wherein the carbon portion of the alkoxy moiety is an n-stearyl moiety.

5. Use according to Claim 1 wherein the carbon portion of the alkoxy moiety is an n-palmityl moiety.

6. Use according to Claim 1 wherein the carbon portion of the aryloxy moiety is a benzyl moiety.

7. Use according to Claim 1 wherein said medicament is for injection intradermally.

8. Use of a composition comprising a derivative of GHL-Cu having the general formula: 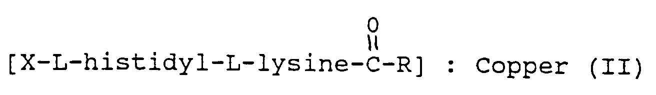 wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for increasing subcutaneous fat in a warm-blooded animal.

9. Use of a composition comprising a derivative of GHL-Cu having the general formula: 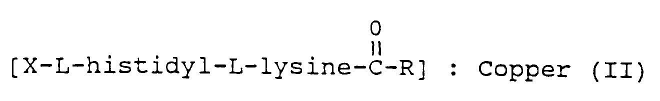 wherein X is glycyl, glycyl-glycyl, L-alanyl, L-seryl or L-valyl R is an alkyl moiety containing from 1 to 18 carbon atoms, an aryl moiety containing from 6 to 12 carbon atoms, an alkoxy moiety containing from 1 to 12, 16 or 18 carbon atoms, or an aryloxy moiety containing from 6 to 12 carbon atoms, or where R is L-prolyl-L-valyl-L-phenylalanyl-L-valine or L-valyl-L-phenylalanyl-L-valine, for the manufacture of a medicament for increasing the density of hair follicles in warm-blooded animals.

10. Use according to any one of Claims 1 to 9 wherein said medicament is for intradermal injection or topical application to a warm-blooded animal.

11. Use according to any one of Claims 1 to 10 wherein the ratio of derivative of GHL to copper (II) is 2:1

## Patentansprüche

1. Verwendung einer Zusammensetzung, die ein Derivat von GHL-Cu umfaßt, mit der allgemeinen Formel: 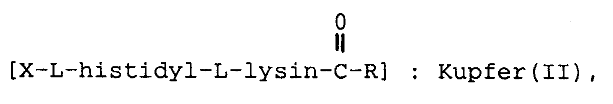 worin X Glycyl, Glycyl-glycyl, L-Alanyl, L-Seryl oder L-Valyl ist, R eine Alkyleinheit, die von 1 bis 18 Kohlenstoff-atome enthält, eine Aryleinheit, die von 6 bis 12 Kohlenstoff-atome enthält, eine Alkoxyeinheit, die von 1 bis 12, 16 oder 18 Kohlenstoffatome enthält, oder eine Aryloxyeinheit ist, die von 6 bis 12 Kohlenstoffatome enthält, oder in der R L-Prolyl-L-valyl-L-phenylalanyl-L-valin oder L-Valyl-L-phenylalanyl-L-valin ist, für die Herstellung eines Medikamentes zur Stimulierung des Haarwachstums bei Warmblütern.

2. Verwendung nach Anspruch 1, wobei der Kohlenstoffteil der Alkoxyeinheit eine unverzweigte Kette ist.

3. Verwendung nach Anspruch 2, wobei die unverzweigte Kette ein n-Octyleinheit ist.

4. Verwendung nach Anspruch 1, wobei der Kohlenstoffteil der Alkoxyeinheit eine n-Stearyleinheit ist.

5. Verwendung nach Anspruch 1, wobei der Kohlenstoffteil der Alkoxyeinheit eine n-Palmityleinheit ist.

6. Verwendung nach Anspruch 1, wobei der Kohlenstoffteil der Aryloxyeinheit eine Benzyleinheit ist.

7. Verwendung nach Anspruch 1, wobei besagtes Medikament für intradermale Injektion ist.

8. Verwendung einer Zusammensetzung, die ein Derivat von GHL-Cu umfaßt; mit der allgemeinen Formel: 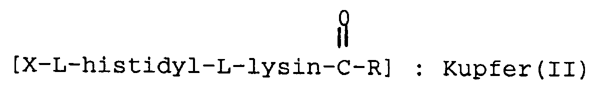 worin X Glycyl, Glycyl-glycyl, L-Alanyl, L-Seryl oder L-Valyl ist, R eine Alkyleinheit, die von 1 bis 18 Kohlenstoff-atome enthält, eine Aryleinheit, die von 6 bis 12 Kohlenstoff-atome enthält, eine Alkoxyeinheit, die von 1 bis 12, 16 oder 18 Kohlenstoffatome enthält, oder eine Aryloxyeinheit ist, die von 6 bis 12 Kohlenstoffatome enthält, oder in der R L-Prolyl-L-valyl-L-phenylalanyl-L-valin oder L-Valyl-L-phenylalanyl-L-valin ist, für die Herstellung eines Medikamentes zur Erhöhung des Unterhautfettes bei einem Warmblüter.

9. Verwendung einer Zusammensetzung, die ein Derivat von GHL-Cu umfaßt; mit der allgemeinen Formel: 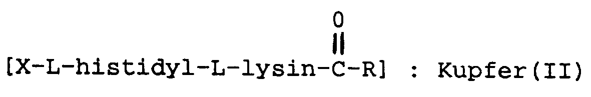 worin X Glycyl, Glycyl-glycyl, L-Alanyl, L-Seryl oder L-Valyl ist, R eine Alkyleinheit, die von 1 bis 18 Kohlenstoff-atome enthält, eine Aryleinheit, die von 6 bis 12 Kohlenstoff-atome enthält, eine Alkoxyeinheit, die von 1 bis 12, 16 oder 18 Kohlenstoffatome enthält, oder eine Aryloxyeinheit ist, die von 6 bis 12 Kohlenstoffatome enthält, oder in der R L-Prolyl-L-valyl-L-phenylalanyl-L-valin oder L-Valyl-L-phenylalanyl-L-valin ist, für die Herstellung eines Medikamentes zur Erhöhung der Haarfollikeldichte bei Warmblütern.

10. Verwendung nach einem der Ansprüche 1 bis 9, wobei besagtes Medikament für intradermale Injektion oder topische Anwendung bei einem Warmblüter ist.

11. Verwendung nach einem der Ansprüche 1 bis 10, wobei das Verhältnis von GHL-Derivat zu Kupfer (II) 2:1 ist.

## Revendications

1. Utilisation d'une composition comprenant un dérivé de GHL-Cu répondant à la formule générale : 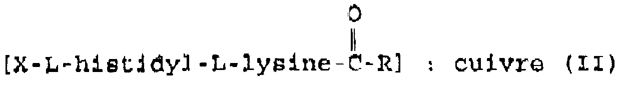 dans laquelle X représente un groupe glycyle, glycyl-glycyle, L-alanyle, L-séryle ou L-valyle, R représente un groupement alkyle contenant 1 à 18 atomes de carbone, un groupement aryle contenant 6 à 12 atomes de carbone, un groupement alkoxy contenant 1 à 12, 16 ou 18 atomes de carbone ou un groupement aryloxy contenant 6 à 12 atomes de carbone, ou dans laquelle R représente un groupe L-propyl-L-valyl-L-phénylalanyl-L-valins ou L-valyl-L-phénylalanyl-L-valine, pour la production d'un médicament destiné à stimuler la croissance des poils chez les animaux à sang chaud.

2. Utilisation suivant la revendication 1, dans laquelle la portion carbonée du groupement alkoxy est une chaîne non ramifiée.

3. Utilisation suivant la revendication 2, dans laquelle la chaîne non ramifiée est un groupement n-octyle.

4. Utilisation suivant la revendication 1, dans laquelle la portion carbonée du groupement alkoxy est un groupement n-stéaryle.

5. Utilisation suivant la revendication 1, dans laquelle la portion carbonée du groupement alkoxy est un groupement n-palmityle.

6. Utilisation suivant la revendication 1, dans laquelle la portion carbonée du groupement aryloxy est un groupement benzyle.

7. Utilisation suivant la revendication 1, dans laquelle le médicament est destiné à l'injection par voie intradermique.

8. Utilisation d'une composition comprenant un dérivé de GHL-Cu répondant à la formule générale : 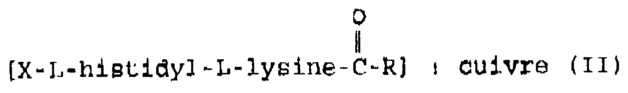 dans laquelle X représente un groupe glycyle, glycyl-glycyle, L-alanyle, L-séryle ou L-valyle, R représente un groupement alkyle contenant 1 à 18 atomes de carbone, un groupement aryle contenant 6 à 12 atomes de carbone, un groupement alkoxy contenant 1 à 12, 16 ou 18 atomes de carbone ou un groupement aryloxy contenant 6 à 12 atomes de carbone, ou bien dans laquelle R représente un groupe L-propyl-L-valyl-L-phénylalanyl-L-valine ou L-valyl-L-phénylalanyl-L-valine, pour la production d'un médicament destiné à augmenter la quantité de graisse sous-cutanée chez un animal à sang chaud.

9. Utilisation d'une composition comprenant un dérivé de GHL-Cu répondant à la formule générale : 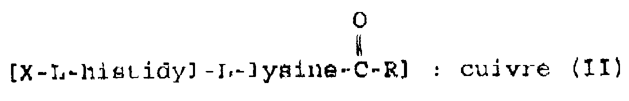 dans laquelle X représente un groupe glycyle, glycyl-glycyle, L-alanyle, L-séryle ou L-valyle, R représente un groupement alkyle contenant 1 à 18 atomes de carbone, un groupement aryle contenant 6 à 12 atomes de carbone, un groupement alkoxy contenant 1 à 12, 16 ou 18 atomes de carbone, ou un groupement aryloxy contenant 6 à 12 atomes de carbone, ou bien dans laquelle R représente un groupe L-propyl-L-valyl-L-phénylalanyl-L-valine ou L-valyl-L-phénylalanyl-L-valine, pour la production d'un médicament destiné à augmenter la densité des follicules pileux chez les animaux à sang chaud.

10. Utilisation suivant l'une quelconque des revendications 1 à 9, dans laquelle le médicament est dostiné à l'injection intradermique ou à l'application topique à un animal à sang chaud.

11. Utilisation suivant l'une quelconque des revendications 1 à 10, dans laquelle le rapport du dérivé de GHL au cuivre (II) est égal à 2:1.
